# EUROPEAN PATENT SPECIFICATION

(11) **EP 0 989 184 B1**
(45) Date of publication and mention of the grant of the patent: **19.11.2008**
(21) Application number: 99118346.8
(22) Date of filing: 16.09.1999
(51) Int. Cl.: C12N 15/00, C12N 5/00, A61K 49/00, A01K 67/027

(54) **A transgenic coagulation factor XIII defective animal and its use for testing wound healing and bleeding**
Transgenes Tier mit einem defekten Gen für den Gerinnungsfaktor XIII sowie dessen Verwendung zum Testen bei Wundheilungen und Blutungen
Animal transgenique comportant un gène de facteur XIII déficient et utilisation dudit animal pour tester la cicatrisation et l'hemorragie

(30) Priority: 23.09.1998 EP 98117978; 16.04.1999 EP 99106270
(43) Date of publication of application: 29.03.2000
(73) Proprietor: CSL Behring GmbH, 35041 Marburg (DE)
(72) Inventor: Dickneite, Gerhard, Dr., 35043 Marburg (DE); Metzner, Hubert, Dr., 35041 Marburg (DE); Zettlmeissl, Gerd, Dr., 35083 Wetter (DE); Grundmann, Ulrich, Dr., 35094 Lahntal (DE); Lathe, Richard, Prof., Edinburgh EH9 3JQ (GB); Smith, Austin, Dr., Edinburgh EH9 3JQ (US); Li, Meng, Dr., Edinburgh EH9 3JQ (US)

(56) References cited:
- EP-A- 0 236 978
- WO-A-91/16931
- WO-A-94/04669
- WO-A-97/46669
- ANWAR, R. ET AL.: "Molecular basis of inherited factor XIII deficiency: identification of multiple mutations provides insights into protein function" BRITISH JOURNAL OF HAEMATOLOGY, vol. 91, 1995, page 728-735 XP002061572 ISSN: 0007-1048
- ICHINOSE, A. ET AL.: "ARG260-cys mutation in severe Factor XIII deficiency: conformational change of the A subunit is predicted by molecular modelling and mechanics" BRITISH JOURNAL OF HAEMATOLOGY, vol. 101, no. 2, May 1998 (1998-05), page 264-272 XP002093671 ISSN: 0007-1048
- MIKKOLA, H. ET AL.: "Deficiency in the A-subunit of coagulation Factor XIII: two novel point mutations demonstrate different effects on transcription levels" BLOOD, vol. 84, no. 2, July 1994 (1994-07), page 517-525 XP002093670 ISSN: 0006-4971
- IZUMI, T. ET AL.: "Novel deletion and insertion mutations cause splicing defects, leading to severe reduction in mRNA levels of the A subunit in severe Factor XIII deficiency" THROMBOSIS AND HAEMOSTASIS, vol. 79, no. 3, March 1998 (1998-03), page 479-485 XP002093669 ISSN: 0340-6245
- ROSIER, F.: "LES SOURIS "KNOCKOUT" ONT LA PÊCHE" LA RECHERCHE, vol. 27, no. 288, June 1996 (1996-06), page 48-50 XP000585254 ISSN: 0029-5671

### SEQUENZPROTOKOLL

<110> Centeon Pharma GmbH
<120> A Transgenic Coagulation Factor XIII Defective Animal and its Use for Testing Wound Healing and Bleeding
<130> C9P11EP
<140>
   <141>
<160> 2
<170> PatentIn Ver. 2.1
<210> 1
   <211> 174
   <212> DNA
   <213> Mus musculus
<400> 1
   gtgaatgcca aggatgatga aggtgttctt gttggatcat gggacaatgt ctatgcctac 60
   ggctcccttc catcagcctg gacaggaagt gttgacattc tactagaata cagaagctcg 120
   gaaacaccag tccgatatgg ccagtgttgg gtttttgctg gtgtctttaa caca       174
<210> 2
   <211> 174
   <212> DNA
   <213> Mus musculus
<400> 2
   gtgaatgcca aggatgacga aggtgttctt gttggatcat gggacaatgt ctatgcctac 60
   ggatcccttc catcagcctg gacaggaagt gttgacattc tactagaata cagaagctca 120
   gaaacaccag tccgatatgg ccagtgctgg gtttttgctg gtgtctttaa caca       174

## Description

This invention relates to a transgenic non-human mammalian animal which is heterozygous or homozygous for an at least partially defective coagulation factor XIII gene.

Factor XIII is an enzyme of the coagulation cascade which cross-links fibrin and thus promotes the stability of the hemostatic plug.

The coagulation is a cascade system of proteolytic enzymes with subsequent activation by limited proteolysis. After wounding the intrinsic and the extrinsic pathways of coagulation are activated. The intrinsic activation includes coagulation factors XII, XI and IX which are proteolytic enzymes (proteases) and an enzyme cofactor (factor VIII). The extrinsic system includes tissue factor and factor VII. Both activation pathways result in the common pathway containing the protease factor Xa and its cofactor (factor Va) which convert the inactive prothrombin (factor II) to the enymatically active thrombin (IIa, n.b.: activated factors are marked with an "a" after the roman number, the roman number alone marks the inactive enzyme). Thrombin is considered to be the central protease of the coagulation system. It cleaves fibrinogen (factor I) resulting in fibrin which is the substrate for the wound closure. Together with blood platelets fibrin forms the hemostatic plug. Thrombin moreover activates the clotting factor XIII by limited proteolysis. In contrast to the other proteases of the coagulation cascade, factor XIII is a transglutaminase. Factor XIII cross-links the fibrin molecule via lysine and glutamine residues, rendering soluble fibrin into insoluble fibrin.

FXIII as found in plasma is composed to two nonidentical subunits, the a-chain and b-chain, with molecular weights of approximately 83.000 and 76.500 Da respectively (McDonagh J., in : Hemostasis and Thrombosis, Colman RW, Hirsh J., March V.J. and Salzmann E.W. (eds), Lippincott, Philadelphia, 1994).

The tetrameric complex in plasma has the composition a₂b₂ with a Mr of 320 000 Da. The concentration of the tetramer in plasma is about 0.07 µmol/l. During the activation step, thrombin cleaves an Arg-Gly bond at the positions 37 and 38 of the a-chain releasing a Mr 4500 activation peptide from the amino terminus. The b-chain dimer dissociates from the complex to release the active a-chain dimer (a₂*, FXIIIa). FXIIIa is a glutamine-lysine transferase. The reaction, catalyzed is the formation of an isopeptide bond between the γ-carbonyl group of glutamine and the ε-amino group of lysine between fibrin molecules, stabilizes the fibrin network. Four to six lysyl-glutamyl crosslinks are formed per mole of fibrin.

The lack of factor XIII in patients might lead to hemorrhagic diathesis and impairment of wound healing (Duckert F., Ann NY Acad Sci, vol. 202, 190-199, 1972). Patients with a congenital FXIII deficiency suffer from soft tissue and joint hemorrhage after trauma. The most serious form of bleeding is in the central nervous system, FXIII deficient patients have a high incidence of intracranial hemorrhage. Women with a FXIII deficiency often undergo abortion during pregnancy. FXIII stimulates the proliferation of fibroblasts, which is essential for wound healing. Thus, patients with FXIII deficiency were reported to have abnormal wound healing.

In this invention the generation of a transgenic coagulation factor XIII defective mouse with disturbance of the wound healing process and bleeding disorders is described. Furthermore, its use is described to develop wound healing accelerating drugs or drugs normalizing coagulation.

A transgenic non-human animal which carries an at least partially defective coagulation factor XIII gene may be prepared by known gene manipulations, in which the factor XIII gene will be inactivated by an insertion, deletion, substitution or inversion or other suitable genetic manipulations. In the performance of the present invention a transgenic non-human animal with a defective coagulation factor XIII gene has been prepared by a targeted disruption of the gene for the factor Xllla subunit with deletion of the active site encoding the exon 7 sequence.

A transgenic mouse has been prepared as follows:

### Preparation of a transgenic mouse

Homologous recombination in embryonic stem (ES) cells was employed to generate mice deficient for factor XIII.

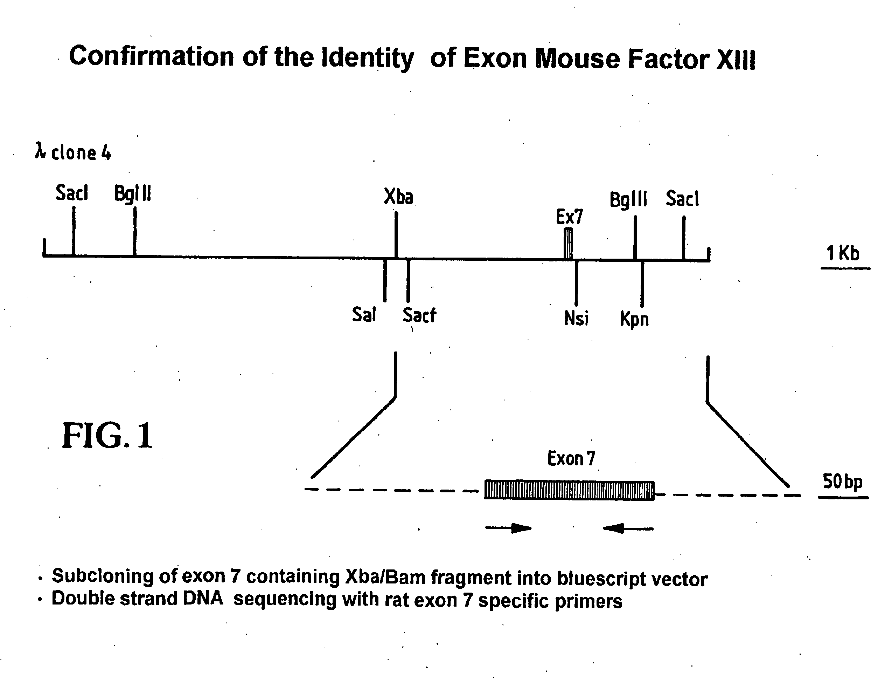

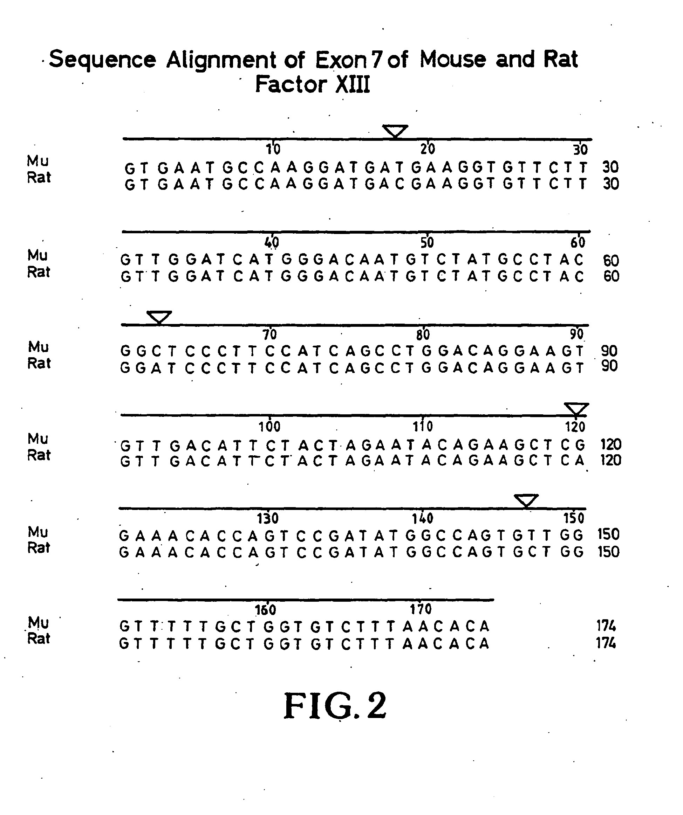

Mouse genomic sequences for the factor XIIIa subunit were isolated from a strain 129 genomic library using rat cDNA sequence as probe. A probe specific for the active site encoding exon (exon 7) was generated by PCR amplification of a 110 bp cognate fragment of the rat cDNA. This probe was used to isolate a 14kb lambda clone (Figure 1). The presence of mouse factor XIII exon 7 sequence was confirmed by DNA sequencing which revealed 170/174 nucleotide identities with the rat exon 7 sequence (Figure 2 and Sequence Listing). The encoded amino acid sequence is completely identical to the rat protein sequence.

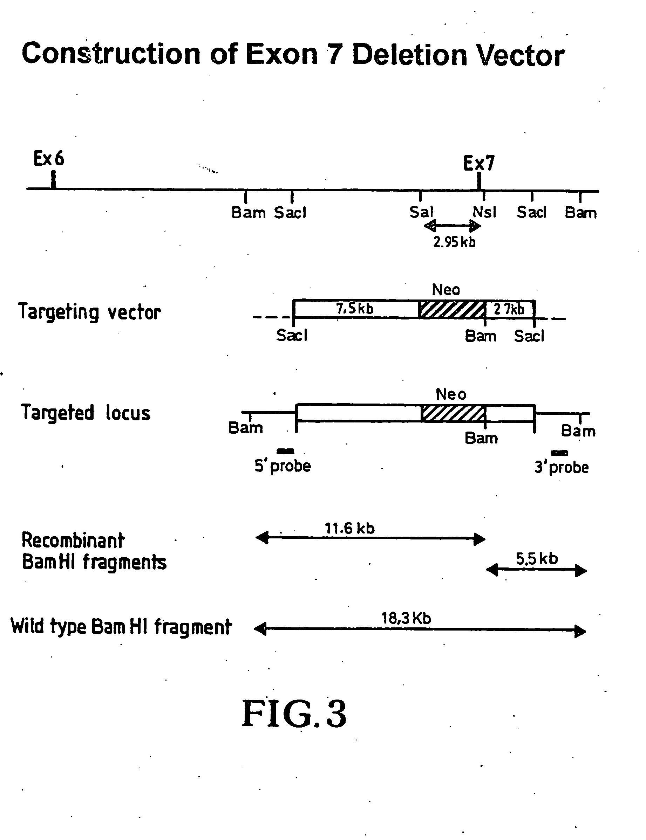

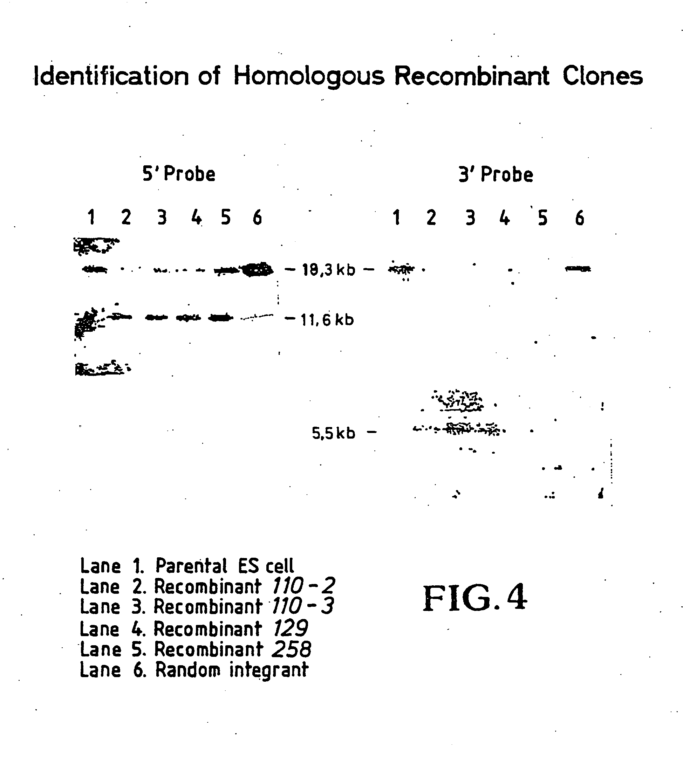

A replacement type targeting vector was constructed using standard recombinant DNA methods. An approximately 2.95kb region of genomic DNA encompassing exon 7 is deleted in the targeting construct and replaced by a b-actin promoter driven neomycin phosphotransferase selectable marker cassette (Figure 3). The targeting construct was digested with Sac 1 to excise the plasmid sequences, then introduced into E14TG2a ES cells by electroporation. Stable transfectants were isolated by selection in G418. Homologous recombinants were identified by Southern hybridisation of genomic DNA with probes external to both 5' and 3' homology regions (Figure 4). From two independent electroporations 4 out of 278 clones analysed gave the predicted hybridisation pattern for the homologous replacement event. Two of these clones (FXIII-110 and FXIII-129) were microinjected into C57BU6 blastocyts to produce chimaeras. Chimaeras were initially test-crossed with outbred Swiss albino mice. Germline transmission was obtained from both clones.

Transmitting chimaeras were crossed with CBA/Ca mice to derive 129Ola x CBA offspring carrying the mutated gene for the Factor XIIIa subunit. Following a backcross generation to CBA/Ca, heterozygous mice were intercrossed. Homozygous animals were identified by Southern hybridisation. The absence of exon 7 sequence in the homozygotes was confirmed using an exon 7 specific probe.

Said mouse has been characterized by its transglutaminase activity.

### Characterization of the factor XIII defective mouse bv its transglutaminase activity

Venous blood was withdrawn and citrate plasma was obtained by centrifugation for further testing of transglutaminase activity. The assay used was the commercially available Berichrom F XIII kit, manufactured by Behring Diagnostics. As reference material human standard plasma has been used. Table 1 shows the results of the plasma transglutaminase determinations of normal mice with the same genetic background but without the defect in the FXIII gene, of hetereozygous FXIII defective mice and of homozgygous FXIII defective mice. The transglutaminase levels of the normal mice determined by Berichrom FXIII was 134%, for the heterozygous deficient animals the level determined was roughly half of that (70%). The transglutaminase levels of the homozygous FXIII defective mice was < 6% . This could be confirmed with another FXIII assay, a clot stability assay where the FXIII activity is determined more specifically by its cross-linking activity towards fibrin which is rendered insoluble in urea or TCA solutions. With this assay no FXIII activity was detectable with a limit of detection of 2%.

**Table 1**

| Transglutaminase levels in plasma of FXIII defective mice and normal control mice | |
|---|---|
| | Transglutaminase activity as % of standard human plasma |
| normal Mice | 134% ± 10.4% |
| heterozygous FXIII defective mice | 70% ± 13.1% |
| homozygous FXIII defective mice | < 6% * |

| | |
|---|---|
| * £ 2% with a clot stability assay (H.E.Karges: Blood Coagulation Factor XIII: determination by clot stability assays. In: Bergmeyer: Methods of Enzymatic Analyses, Third Ed. Vol.V; Verlag Chemie, Weinheim, 1984; pp 400-410) | |

### - Use of the FXIII defective mouse - as a model for impaired wound healing and bleeding

FXIII defective mice were anaesthetized with hexobarbital and the dorsal area was shaved. A full thickness midline incision of the skin was made with a surgical blade along the spine. The wound was then closed with clips and the mice were allowed to wake up from anaesthesia.

Two days later the clamps were removed. At 7, 9, and 16 days after wounding the tensile strength of the healed wound was investigated for each animal.

Mice were sacrified and the dorsal skin was removed. The skin was then cut into pieces of exactly one centimeter width and 3 centimeter length with centrally located incisions. The skin piece was then introduced into a tensiometer (Hounsfield Test Equipment, Salford, Redhill, England) and the tensile strength of the incision wound was measured according to the method described by Mustoe et. a. (Science, vol. 237, 1333-1336, 1987). A force was applied across the incision by tearing with a constant speed. The force was recorded graphically and the breaking strenght was defined as the point of maximum stress before wound separation.

The breaking strength was given in Newton (N). As a control we used a mouse strain with the same genetic background without the inserted defect gene normal mice), which underwent the same experimental procedure.

Table 2 shows the results of the wound healing study. Compared to the control there was lower breaking strength in the FXIII defective mice when compared to normal mice. This clearly indicates an impaired wound healing.

**Table 2**

| Breaking strength of skin pieces from FXIII defective mice and normal control mice (Strain CBA) | | |
|---|---|---|
| | normal mice (N) | FXIII defective mice (N) |
| day 7 | 1.03 ± 0.09 | 0.69 ± 0.11 |
| day 9 | 2.3 ± 0.22 | 1.46 ± 0.14 |
| day 16 | 5.2 ± 0.03 | 3.3 ± 0 |

The test of the bleeding time serves as a measure of the hemostatic function. For the bleeding time the tail was resected at about 2 mm apart from the tip (tail tip bleeding). The time until cessation of bleeding was monitored by blotting the blood from the wound with a filter paper. Bleeding time was given in seconds for termination of bleeding. Table 3 denotes the results of the bleeding time. FXIII defective mice had a significantly prolonged bleeding time in comparison to normal mice.

**Table 3**

| Breaking strength of skin pieces from FXIII defective mice and normal control mice (Strain CBA) | |
|---|---|
| | Bleeding time (sec.) |
| normal mice | 170 ± 3 |
| FXIII defective mice | 274 ± 50 |

## Claims

1. A transgenic non-human mammalian animal which is heterozygous or homozygous for an at least partially defective coagulation factor XIII gene.

2. A transgenic animal according to claim 1 in which the defective gene has been inactivated by an insertion, deletion, substitution or inversion or other suitable genetic manipulation.

3. A transgenic animal according to claim 1 or 2 in which the defective coagulation factor XIII gene has been prepared by a targeted disruption of the gene for the factor XIIIa with deletion of the active site encoding exon 7 sequence.

4. A transgenic animal according to claim 1 to 3 which is a rodent.

5. A transgenic animal according to claim 4 which is a mouse.

6. A transgenic factor XIII defective mouse **characterized by** the deletion of the factor XIIIa exon sequence.

7. A method of testing the pharmacological properties of a compound in relation to its coagulation factor XIII activity, the method comprising contacting a transgenic animal as claimed in claims 1 to 6 with said compound and monitoring its effect on disturbances of the wound healing process and bleeding disorders.

8. Use of transgenic animal as claimed in claims 1 to 6 for the investigation of orally or parenterally administered pharmaceuticals on wound healing, bleeding or other diseases associated with a deficiency in factor XIII.

## Patentansprüche

1. Transgenes nicht-humanes Säugetier, welches für ein zumindest partiell defektes Gen für den Gerinnungsfaktor XIII heterozygot oder homozygot ist.

2. Transgenes Tier nach Anspruch 1, worin das defekte Gen durch eine Insertion, Deletion, Substitution oder Inversion oder eine andere gentechnische Manipulation verändert worden ist.

3. Transgenes Tier nach Anspruch 1 oder 2, worin das defekte Gen für den Gerinnungsfaktor XIII durch gezielte Disruption des Gens für den Faktor XIIIa mittels Deletion der Exon 7-Sequenz, die das aktive Zentrum codiert, hergestellt worden ist.

4. Transgenes Tier nach Anspruch 1 bis 3, bei dem es sich um ein Nagetier handelt.

5. Transgenes Tier nach Anspruch 4, bei dem es sich um eine Maus handelt.

6. Transgene Maus, die für den Faktor XIII defizient ist, **gekennzeichnet durch** die Deletion der Faktor XIIIa-Exonsequenz.

7. Verfahren zum Überprüfen der pharmakologischen Eigenschaften einer Verbindung in Bezug auf ihre Gerinnungsfaktor XIII-Aktivität, wobei das Verfahren das Zusammenbringen eines transgenen Tiers nach den Ansprüchen 1 bis 6 mit der Verbindung und das Überwachen ihrer Wirkung auf Störungen des Wundheilungsprozesses und Blutungsstörungen umfasst.

8. Verwendung eines transgenen Tiers nach den Ansprüchen 1 bis 6 zur Untersuchung oral oder parenteral verabreichter Arzneistoffe auf die Wundheilung, Blutung oder andere Krankheiten, die mit einer Defizienz des Faktors XIII einhergehen.

## Revendications

1. Animal transgénique mammalien non humain qui est hétérozygote ou homozygote pour un gène au moins partiellement défectueux du facteur XIII de la coagulation.

2. Animal transgénique selon la revendication 1, dans lequel le gène défectueux a été inactivé par une insertion, une délétion, une substitution ou une inversion, ou une autre manipulation génétique appropriée.

3. Animal transgénique selon la revendication 1 ou 2, dans lequel le gène défectueux du facteur XIII de la coagulation a été préparé par une disruption ciblée du gène du facteur XIIIa avec une délétion de la séquence de l'exon 7 codant pour le site actif.

4. Animal transgénique selon les revendications 1 à 3, qui est un rongeur.

5. Animal transgénique selon la revendication 4, qui est une souris.

6. Souris transgénique déficiente en facteur XIII, **caractérisée par** la délétion de la séquence exonique du facteur XIIIa.

7. Procédé permettant de tester les propriétés pharmacologiques d'un composé relativement à son activité de facteur XIII de la coagulation, le procédé consistant à mettre un animal transgénique selon les revendications 1 à 6 en contact avec ledit composé et à suivre son effet sur les perturbations du processus de cicatrisation des plaies et les troubles de saignement.

8. Utilisation de l'animal transgénique selon les revendications 1 à 6, pour l'étude de produits pharmaceutiques administrés par voie orale ou parentérale sur la cicatrisation des plaies, le saignement ou d'autres maladies associées à une déficience en facteur XIII.
